# EUROPÄISCHE PATENTSCHRIFT

(11) **EP 1 711 216 B1**
(45) Veröffentlichungstag und Bekanntmachung des Hinweises auf die Patenterteilung: **02.07.2008**
(21) Anmeldenummer: 05714930.4
(22) Anmeldetag: 03.02.2005
(51) Int. Cl.: A61M 5/145, A61M 5/20, A61M 5/48, A61M 5/168

(54) **ANÄSTHESIESPRITZE**
ANESTHESIA SYRINGE
SERINGUE POUR ANESTHESIE

(30) Priorität: 03.02.2004 DE 102004005383; 04.02.2004 US 541811 P; 07.05.2004 DE 102004023235
(43) Veröffentlichungstag der Anmeldung: 18.10.2006
(73) Patentinhaber: SMJM Inject GmbH, 52062 Aachen (DE)
(72) Erfinder: MANSOURI, Said, 52062 Aachen (DE)
(74) Vertreter: Castell, Klaus
(86) Internationale Anmeldenummer: PCT/DE2005/000178
(87) Internationale Veröffentlichungsnummer: WO 2005/075009

(56) Entgegenhaltungen:
- WO-A-02/49697
- WO-A-03/041765
- DE-A1- 19 614 337
- US-A- 2 650 591
- US-A- 4 710 172

## Beschreibung

Die Erfindung betrifft eine Anästhesiespritze.

In vielen Bereichen der Medizin stellt sich regelmäßig die Aufgabe, Anästhesiemittel in einen menschlichen oder tierischen Körper zu injizieren. Hierzu werden Anästhesiespritzen verwendet, die sich auch im Wesentlichen bewährt haben.

Der weit überwiegende Teil der in der Praxis verwendeten Anästhesiespritzen ist manuell zu bedienen. Diese Art der Anästhesiespritzen bedarf auf Grund ihrer allgemeinen Bekanntheit keiner Erläuterung.

Um den Injektionsvorgang mit einer Anästhesiespritze verbessert steuerbar und wiederholbar zu machen, wurden jedoch auch vollautomatische und halbautomatische Anästhesiespritzen vorgeschlagen. So offenbart die DE 196 43 813 A1 einen Bausatz für eine Spritze, bei welcher eine Regelelektronik vorgesehen ist, die geeignet ist, den Medikamentenfluss zu errechnen. Für die Rückmeldung über den Vorschub ist eine fotooptische Ablesung vorgesehen.

Die DE-Auslegeschrift 25 00 851 zeigt einen Injektor für ein Kontrastmittel, wobei der Injektor den Durchfluss, die zu injizierende Gesamtmenge und den Druck des Kontrastmittels über eine Regelelektronik steuert.

Eine hierzu ähnliche Vorrichtung zeigt die WO 01/1397 A2, wobei die Steuerungseinheit im Wesentlichen getrennt von der Injektionsspritze ist und der Injektionsdruck über ein Fußpedal kontrolliert wird.

Diese Systeme sind jedoch alle äußerst aufwendig und unhandlich. Als Verbesserung schlägt die EP 0 567 186 B1 eine handgehaltene Vorrichtung zum Ausdrücken von Injektionsspritzen vor, bei welcher ein Kolben über eine motorgetriebene Gewindestange durch einen Carpulenraum vorgeschoben wird. Zum Antrieb des Motors ist lediglich ein Stromkabel zu einem Netzanschluss zu führen.

Die US 5,108,317 schlägt eine Anästhesiespritze vor, bei welcher von einer externen Steuerungseinheit über eine Hydraulikleitung zwei Kammern in der Spritze wechselweise gefüllt beziehungsweise entleert werden. Die Kammern befinden sich auf der Vorderseite beziehungsweise auf der Rückseite einer Kolbenplatte, wodurch ein mit der Kolbenplatte verbundener Vorschubkolben durch einen Carpulenraum geführt wird und den Inhalt der Carpule zur Injektion durch eine Nadel ausführt. Hierdurch soll ebenfalls ein besonders kontrolliertes Ausstoßen des Anästhetikums erfolgen, was zu einer Reduzierung der erforderlichen Gesamtmenge am Anästhetikum führen soll.

Mit dem gleichen Ziel schlägt die US 5,690,618 eine elektronische Anästhesiespritze vor, wobei ein elektronischer Antrieb mit einer externen Energiequelle in einem kugelschreiberähnlich geformten Spritzengehäuse angeordnet ist, was zu einem besseren Handling der Spritze führen soll.

Als bislang weitestgehende Entwicklung wird die WO 03/041 765 A1 angesehen. Die hier vorgeschlagene Anästhesiespritze ist ebenfalls im Wesentlichen kugelschreiberförmig gestaltet und somit leicht zu bedienen. Sie ist jedoch autark funktionstüchtig, also unabhängig von einer externen Energiequelle. Hierzu wird eine Gasdruckpatrone an die Spritze geschraubt, welche über einen mit der Patrone verbundenen Faltenbalg in einer rückwärtigen Hydraulikkammer einen Antriebsdruck erzeugt. Nach Öffnen eines Verschlusses in einem Hydraulikkanal erstreckt sich dieser Druck auch auf eine vordere Hydraulikkammer, welche einen Vorschubkolben umgibt. An einer Kolbenplatte entsteht eine resultierende Vortriebskraft entsprechend der Größe der Kolbenstange. Hierdurch wird der Kolben durch einen Carpulenraum geschoben und ein Anästhetikum aus der Spritze ausgedrückt. Die vordere Hydraulikkammer sorgt bei geschlossenem Hydraulikkanal dafür, dass der Kolben nicht aus der Spritze herausgezogen werden kann.

Diese Anästhesiespritze ist jedoch relativ aufwendig in der Fertigung. Insbesondere benötigt sie mehrere kostspielige Dichtungen und einen diffizil herauszuarbeitenden Hydraulikkanal zur Verbindung der beiden Hydraulikkammern vor und hinter der Kolbenplatte.

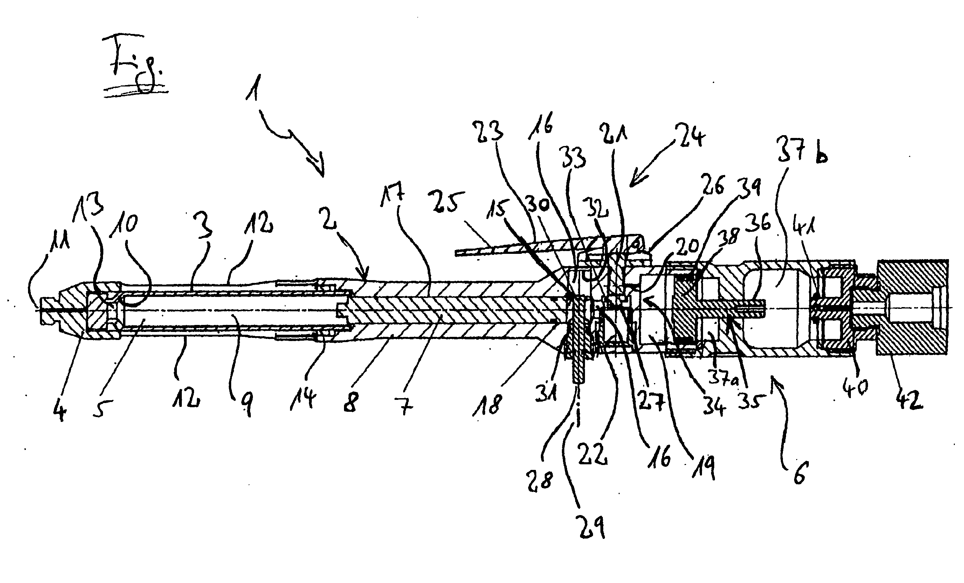

Die WO 02/49697 A1 zeigt eine Anästhesiespritze mit Hydraulikkammern und mit einem Ventilschieber. So ist in der dortigen Figur 1A eine solche Spritze im Längsschnitt dargestellt, bei welcher der Ventilschieber, gesteuert durch einen Benutzerknopf, in einer Trennwand zwischen den beiden Hydraulikkammern angeordnet ist, wobei die eine Hydraulikkammer rückwärtig von einem Antriebskolben beaufschlagt wird, während die andere Hydraulikkammer bei Steigerung des Drucks einen Kolben ausschiebt, der zum Ausspritzen des in der Anästhesiespritze enthaltenen Fluids führt. Der mit dem Benutzerknopf verbundene Ventilschieber sitzt in einer Kammer, die eine Feder aufnimmt, um für den Benutzerknopf eine Rückstellkraft bereits zu stellen. Die Spritze ist so aufgebaut, dass sowohl dieser Raum, der die Feder aufnimmt, als auch der Raum zwischen dem Benutzerknopf und dem zylindrischen Ventilschieber von Hydraulikflüssiglceit freigehalten wird. Der Flüssigkeitsstrom zwischen den beiden Hydraulikkammern wird durch die Zylindermantelfläche des Ventilschiebers geregelt.

Weitere Spritzen und ähnliche Vorrichtungen oder Verfahren sind aus der US 2004/0055662 A1, aus der US 6,440,099 B2, aus der US 2002/0055712 A1, aus der US 4,710,172, aus der US 2,650,591, aus der WO 02/081009 A2, aus der DE 196 14 337 A1, aus der US 2003/0105433 A1, aus der US 2003/0195477 A1 und aus der US 5,730,723 bekannt.

Der vorliegenden Erfindung liegt die Aufgabe zu Grunde, eine verbesserte Spritze zur Verfügung zu stellen.

Diese Aufgabe wird gelöst mit einer Anästhesiespritze mit den Merkmalen des Hauptanspruchs. Die Merkmale der Unteransprüche führen zu vorteilhaften Ausführungsvarianten.

Unter einer Anordnung der zweiten Hydraulikkammer "rückwärtig" der ersten Hydraulikkammer sei verstanden, dass die zweite Hydraulikkammer nicht am Vorschubkolben - und in Vorschubrichtung vor der Kolbenplatte - angeordnet ist, sondern vielmehr seriell mit der ersten Hydraulikkammer angeordnet und durch diese vom Vorschubkolben getrennt ist. Somit verkörpert sich ein Aspekt der Erfindung in einer Umlagerung des hydraulischen Systems zum Vortrieb und zur Regelung des Vortriebs. Vorteilhaft steht so ein erheblich größerer Druck zum Vortrieb des Vorschubkolbens zur Verfügung. Versuche haben ergeben, dass die Hydraulikkammer zum Antrieb des Vorschubkolbens Drücke von bis zu 150 bar ausüben muss, um eine besonders große Bandbreite an Anwendungsmöglichkeiten der Anästhesiespritze zu ermöglichen. Bei der vorgeschlagenen Anordnung der Hydraulikkammern tritt an der Druckplatte des Vorschubkolbens kein Gegendruck auf, wodurch der gesamte Druck der ersten Hydraulikkammer zur resultierenden Vorschubkraft des Vorschubkolbens genutzt werden kann. Außerdem kann die Spritze schlanker ausgeführt werden, weil das Erfordernis einer Hydraulikleitung neben der Kolbenplatte entfällt.

Gemäß einem Aspekt der Erfindung weist die erfindungsgemäße Anästhesiespritze einen Ventilschieber auf, welcher eine Öffnung einer Steuerbohrung zwischen der ersten Hydraulikkammer und der zweiten Hydraulikkammer schließen oder stufenlos öffnen kann. Die Verbindung zwischen der ersten und der zweiten Hydraulikkammer eignet sich besonders, um die resultierende Bewegung am Vorschubkolben fein dosiert regulieren zu können, wenn die zweite Hydraulikkammer unter großem Überdruck steht. Mit einem Ventilschieber, bevorzugt mit zumindest im Wesentlichen linearer Schließcharakteristik, geschieht dies auf besonders effektive Weise.

Um eine haptische Rückkopplung des Drucks zu ermöglichen, welcher durch Öffnen des Ventilschiebers in der ersten Hydraulikkammer aufgebaut ist, wird vorgeschlagen, dass der Ventilschieber eine Druckplatte hat, die an die erste Hydraulikkammer angeschlossen ist. Insbesondere kann der Ventilschieber mit einem Stirnelement in die erste Hydraulikkammer hineinragen.

Wesentliche Eigenschaft eines Ventilschiebers ist, dass er längs einer Bewegungsachse verschoben werden kann, wobei abhängig von seiner Position entlang der Bewegungsachse ein Ventildurchlass in der Steuerbohrung mehr oder weniger geöffnet wird. Sobald der Ventilschieber an die erste Druckkammer angeschlossen ist beziehungsweise nur mit einem Teil in diese hineinragt, entsteht beim Aufbau eines Drucks in der ersten Hydraulikkammer eine hydrostatische Kraft, welche den Ventilschieber entlang seiner Bewegungsachse aus der ersten Hydraulikkammer hinausdrückt. Somit kann an der Kraft, mit welcher der Ventilschieber aus der Hydraulikkammer hinausgedrückt wird, der Druck in der ersten Hydraulikkammer erkannt werden. Wenn die Anästhesiespritze einen Taster hat, der durch Eindrücken einer Tastfläche - zumindest im Wesentlichen - parallel zu der Bewegungsachse des Ventilschiebers die Steuerbohrung öffnet, lässt sich der vorhandene Druck in der ersten Hydraulikkammer unmittelbar an der Gegenkraft, welche gegen das Eindrücken der Tastfläche wirkt, haptisch erkennen.

Es sei betont, dass dieser Vorteil auch bei einer Anordnung der beiden Hydraulikkammern nebeneinander in der Spritze, aber für beide Hydraulikkammern rückwärtig des Vorschubkolbens, gewährleistet sein kann. Auch eine solche Anordnung ist unabhängig von sämtlichen übrigen Merkmalen der vorliegenden Erfindung vorteilhaft und erfinderisch.

In einer bevorzugten Ausführungsform der erfindungsgemäßen Spritze befindet sich rückwärtig der zweiten Hydraulikkammer ein Trennkolben, welcher die zweite Hydraulikkammer verkleinerbar verschieblich gelagert ist. Das bedeutet, dass die zweite Hydraulikkammer durch entsprechendes Verschieben des Trennkolbens verkleinert werden kann.

Über einen Trennkolben kann auf einfache und gleichzeitig variable Weise ein Antriebsdruck in der zweiten Hydraulikkammer erzeugt werden. Beispielsweise kann der Trennkolben in der Anästhesiespritze über eine starke Feder mit ständiger Druckausübung auf die zweite Hydraulikkammer gelagert sein.

Insbesondere wird jedoch vorgeschlagen, dass der Trennkolben in einen Druckraum ragt und diesen vergrößerbar verschieblich gelagert ist, wobei eine den Druckraum vergrößernde Verschiebung des Trennkolbens eine Verkleinerung der zweiten Hydraulikkammer bewirkt. Hierdurch wird es möglich, das hydraulische System getrennt von einem weiteren Druckraum zu betreiben, wobei der Druckraum mit einer Flüssigkeit oder einem Gas befüllt sein kann. Der Druckraum lässt sich besonders einfach ausführen, wenn ein Gas in diesem gespeichert werden soll. Gase sind gegenüber Flüssigkeiten erheblich besser komprimierbar. Somit kann ein im Druckraum vorhandenes, unter Druck stehendes Gas über den Trennkolben auf das hydraulische System, insbesondere auf die zweite Hydraulikkammer, wirken. Der Druckraum ist somit als Gasfeder verwendbar.

Bevorzugt ist der Trennkolben rückverschieblich - den Druckraum verkleinernd und gleichzeitig die zweite Hydraulikkammer vergrößernd - gelagert. In dieser Form ist die Spritze einer mehrfachen Verwendung unter wiederholter Nutzung der Federkraft im Druckraum zugänglich.

Es ist von Vorteil, wenn bei einer Vergrößerung des Druckraums durch Verschiebung des Trennkolbens die zweite Hydraulikkammer um ein gleiches Maß verkleinert wird. Bei ausreichender Vorspannung des komprimierten Gases im Druckraum kann dieses den Trennkolben relativ weit ausschieben, ohne dass der Druck im Druckraum zu sehr absinkt. Bei einer Gestaltung der Verdrängungsverhältnisse wie vorgeschlagen - also mit einem hydraulischen Übersetzungsverhältnis von 1:1 - kann der Trennkolben besonders einfach ausgeführt werden. Insbesondere bietet sich eine einfache Scheibe an, welche gleichzeitig den Druckraum und die zweite Hydraulikkammer begrenzt und zwischen diesen frei verschieblich ist. Dabei kann am Rand der Scheiben vorzugsweise eine Doppeldichtung vorgesehen sein, um das hydraulische System vom pneumatischen System sicher zu trennen.

Um den Druckraum mit Gas unter Überdruck füllen zu können, wird vorgeschlagen, dass der Druckraum einen Anschluss für eine Druckgaszufuhr, insbesondere eine Aufnahme für eine Gaskartusche, aufweist.

Die Spritze kann besonders gut gegriffen und gleichzeitig ein Taster bedient werden, wenn dessen Tastfläche zumindest zum Teil in einer vorderen Hälfte der Anästhesiespritze angeordnet ist. Die vorgeschlagene Anästhesiespritze zeichnet sich nach einem Aspekt der Erfindung durch eine hervorragende Handlichkeit aus. Dies kann auch dadurch erzeugt werden, dass die Spritze - einem Kugelschreiber ähnlich - weit vorne gegriffen und bedient werden kann. Auf diese Weise kann der Arzt die Nadel relativ direkt führen, während ein Teil der Spritze nach hinten aus seiner Hand hinausragt und beim Setzen der Spritze nicht stört. Dies kann insbesondere dann von Vorteil sein, wenn der Arzt einen erhöhten Einstechwiderstand zu überwinden hat und die Spritze mit entsprechend hohen Kräften nach vorne drücken muss.

Um den Einspritzdruck unabhängig von der entlang der Längserstreckungsrichtung der Spritze ausgeübten Kraft spürbar zu machen, wird vorgeschlagen, dass eine Bewegungsachse des Ventilschiebers zumindest im Wesentlichen senkrecht zu einer Längserstreckungsrichtung der Spritze angeordnet ist. Bei einer im Wesentlichen runden Spritzenform kann der Ventilschieber insbesondere radial angeordnet sein.

Um unabhängig vom hydraulischen Druck in der ersten Hydraulikkammer gewährleisten zu können, dass die Spritze ohne bewusste Betätigung keinen Vorschub leistet, wird vorgeschlagen, dass Ventilschieber mit einer die Steuerbohrung schließenden Vorspannkraft beaufschlagt ist. Hierzu kann beispielsweise eine herkömmliche Spiralfeder am Ventilschieber angeschlossen sein.

In einer vorteilhaften Ausführungsvariante der erfindungsgemäßen Anästhesiespritze hat diese einen Indexkolben, der an der ersten Hydraulikkammer angeschlossen ist. Ein Indexkolben ermöglicht es, den Druck in der ersten Hydraulikkammer alternativ oder kumulativ zu einer haptischen Rückkopplung auch optisch erkennbar zu machen. Hierzu kann der Indexkolben insbesondere mit einem Fuß in die erste Hydraulikkammer hineinragen, so dass bei einem Druckaufbau in der ersten Hydraulikkammer eine resultierende Kraft auf den Indexkolben wirkt, welche diesen aus der ersten Hydraulikkammer hinaustreibt. Wenn der Indexkolben zumindest teilweise aus dem Gehäuse der Spritze hinausragend verschieblich gelagert ist, wobei vorzugsweise ein Austrittsbegrenzer vorgesehen ist, kann unmittelbar am Ausschub des Indexkolbens aus dem Gehäuse der Anästhesiespritze der Druck im Inneren erkannt werden. Um den Druck in der ersten Hydraulikkammer auch größenmäßig ablesen zu können, wird vorgeschlagen, dass der Indexkolben gegen eine Austrittsrichtung vorgespannt gelagert ist, wobei sich insbesondere die Vorspannung über eine herkömmliche Spiralfeder eignet, da über die Federkonstante der Verschiebungsweg des Indexkolbens und der hierzu aufgebrachte Druck proportional gekoppelt sind.

Damit nur bestimmte, für die erfindungsgemäße Spritze vorgesehene Kanülen mit der Spritze verwendet werden, wird vorgeschlagen, dass die Spritze eine Spezialaufnahme für eine Spezialkanüle aufweist. Dies kann beispielsweise ein spezielles Gewinde, ein spezieller Bajonettverschluss oder eine spezielle Rasteinrichtung sein, welche die Spezialkanüle so aufnimmt, dass diese eine Fluidleitende Verbindung zur Glascarpule erhält.

Damit der Vorschubkolben nicht unbeabsichtigt - unter Ziehen eines Unterdrucks - aus dem Spritzengehäuse gezogen werden kann, wird vorgeschlagen, dass der Vorschubkolben in einer inneren Lage vollständig in einem Vorschubzylinder aufgenommen ist. Somit kann bei in die Anästhesiespritze zurückgezogenem Druckkolben die Zylinderwand die Spitze des Vorschubkolbens abdecken, sodass diese nicht mehr gegriffen werden kann. An der vorn überstehenden Zylinderwand kann der Anschluss für den Carpulenraum angeordnet sein.

Es sei darauf hingewiesen, dass eine Anästhesiespritze, bei welcher ein Trennkolben in einem Druckraum und in einer zweiten Hydraulikkammer bei wechselseitig volumenverändernder Verschieblichkeit liegt, auch für sich genommen vorteilhaft und erfinderisch ist. Gleiches gilt für eine solche Anästhesiespritze, bei welcher zusätzlich das Volumen des Druckraums bei Verschiebung des Trennkolbens um ein gleiches Maß wie die zweite Hydraulikkammer verändert wird. Dies kann besonders einfach durch einen Trennkolben bewerkstelligt werden, welcher im Druckraum einen gleichen Durchmesser hat wie in der zweiten Hydraulikkammer.

Auch sei betont, dass eine Anästhesiespritze mit hydraulischem oder pneumatisch-hydraulischem Antrieb, bei welcher ein Ventilschieber für eine haptische Rückkoppelung ausgelegt ist, ebenfalls unabhängig von den übrigen Merkmalen vorteilhaft und erfinderisch ist. Gleiches gilt im übrigen für eine Anästhesiespritze mit einem Indexkolben, der an der ersten oder der zweiten Hydraulikkammer angeschlossen ist.

Die Erfindung wird nachstehend anhand eines Ausführungsbeispiels in der Zeichnung näher erläutert. Hier zeigt die einzige
- Figur: eine Anästhesiespritze mit einer seriellen Anordnung aus Carpulenraum, Vorschubkolben, erster Hydraulikkammer, zweiter Hydraulikkammer, Trennkolben und Druckraum.

Die Anästhesiespritze 1 in der Figur besteht im Wesentlichen aus einem Gehäuse 2, welches in einem Carpulenraum 3 einer Carpulenhülse 4 eine Carpule 5 aufnehmen und deren Inhalt mit einem hydraulischpneumatischen Antriebssystem 6 über einen Vorschubkolben 7 austreiben kann.

Der Vorschubkolben 7 ist in einem Vorschubzylinder 8 entlag einer Haupterstreckungsachse 9 der Anästhesiespritze 1 verschieblich gelagert, wobei der Vorschubkolben 7 in der Figur eine innere Endlage einnimmt und bei einem Vorschub entlag der Achse 9 bis zu einer Anschlagschulter 10 bewegt werden kann, wo er eine äußere Endlage einnimmt. Beim Vorschub durchfährt der Vorschubkolben 7 den Carpulenraum 3, so dass Flüssigkeit aus einer eingelegten Carpule 5 durch eine Nadelöffnung 11 ausgespritzt werden kann. Zum Einsatz der Spritze 1 wird in die Nadelöffnung 11 eine Kanüle eingesetzt. Die Kanüle hat eine Spitze zum Einstechen ins Gewebe und eine zweite Spitze zum Einstechen in die Dichtmembran der eingelegten Carpule 5.

In der Nadelöffnung 11 ist eine Spezialaufnahme für die Kanüle vorgesehen, (nicht im Detail dargestellt), damit nicht versehentlich eine Kanüle in die Spritze 1 eingesetzt werden kann, welche bei den mitunter hohen Drücken mechanisch versagt.

Die Carpulenhülse 4 hat zwei Sichtfenster 12, durch welche die Vorschubposition des Vorschubkolbens 7 innerhalb der Carpulenhülse 4 einsehbar ist. Die Carpule 5 hat eine handelsübliche Kopfgestaltung 13, so dass der behandelnde Arzt nicht in der Wahl der Carpulen eingeschränkt wird, welche er bereits von der Arbeit mit herkömmlichen Spritzen kannte. Die Carpulenhülse 4 ist über einen Bajonettverschluss 14 mit dem Vorschubzylinder 8 fest verbunden.

Mit einer Druckplatte 15 ist der Vorschubkolben 7 einer ersten Hydraulikkammer 16 zugewandt, wobei eine Mantelfläche 17 des Vorschubkolbens 7 über einen O-Ring 18 gegenüber der ersten Hydraulikkammer 16 abgedichtet ist.

Rückwärtig der ersten Hydraulikkammer 16 ist eine zweite Hydraulikkammer 19 vorgesehen und mit der ersten Hydraulikkammer 16 über eine Steuerbohrung 20 verbunden. Die Öffnung der Steuerbohrung 20 zwischen der ersten Hydraulikkammer 16 und der zweiten Hydraulikkammer 19 regelt ein Ventilschieber 21, welcher entlang einer Bewegungsrichtung 22 verschieblich gelagert ist und an einer Außenseite des Gehäuses 2 an einen Tasterarm 23 eines Steuertasters 24 stößt. Dem vorderen Bereich der Spritze 1 zugewandt weist der Steuertaster 24 eine Tastfläche 25 zum Niederdrücken des Tasterarms 23 - und somit des Ventilschiebers 21 - auf. Hierzu ist der Tasterarm 23 an einem Tasterlager 26 um einen Tasterbolzen drehbar gelagert. Der Tasterbolzen liegt senkrecht zur Haupterstreckungsachse 9 der Anästhesiespritze 1.

Der Ventilschieber 21 ragt mit einer Druckplatte 27 in die erste Hydraulikkammer hinein, während die Mantelfläche des Ventilschiebers 21 mit einem O-Ring abgedichtet ist.

In ähnlicher Weise ist ein Indexkolben 28 im Gehäuse 2 entlang einer zur Haupterstreckungsrichtung 9 der Spritze 1 radialen Richtung 29 gelagert. Der Indexkolben 28 ragt mit einem Fuß 30 in die erste Hydraulikkammer 16 hinein, während die Mantelfläche des Indexkolbens 28 mit einem O-Ring 31 abgedichtet ist. Eine Druckfeder (nicht dargestellt) presst den Indexkolben 28 zu einer inneren Endlage, bei welcher der Fuß 30 an einer inneren Begrenzungsfläche 32 zum Liegen kommt (in der Figur ist der Indexkolben 28 zur besseren Übersichtlichkeit bis zu einem Anschlag ausgefahren dargestellt; ohne Druck in der Hydraulikkammer 1 nimmt der Indexkolben bei der vorliegenden Ausführung 1 jedoch in der Praxis die innere Endposition ein).

Eine weitere Druckfeder 33 presst den Ventilschieber 21 unter Vorspannung im Ruhezustand in die dargestellte äußere Endlage, welche durch Anschlag der gegenüber dem zylindrischen Teil des Schiebers 21 vergrößert ausgeführten Druckplatte 27 an das Gehäuse 2 an einem Anschlag 34 definiert wird.

Die zweite Hydraulikkammer 19 ist rückwärtig von einem Trennkolben 35 begrenzt, welcher entlang der Haupterstreckungsrichtung 9 der Spritze 1 verschieblich gelagert ist und seinerseits rückwärtig in einen Druckraum 37a, 37b ragt und diesen somit begrenzt. Eine Druckplatte 38 bildet das wesentliche Element des Trennkolbens 35. An Seiten der Druckplatte 38 ist eine Doppel-O-Ring-Dichtung 39 vorgesehen. Damit der Trennkolben 35 bei einer Bewegung zwischen der zweiten Hydraulikkammer 19 und dem Druckraum 37a nicht verkantet, ist im Druckraum 37, 37b eine Führung für einen zylindrischen Führungsstab 36 des Trennkolbens 35 vorgesehen. Große Durchlässe verbinden die beiden Teilkammern 37a und 37b des Druckraums (in der Figur nicht dargestellt).

Der Druckraum 37b wird rückwärtig durch einen Verschlussstopfen 40 begrenzt, welcher über einen weiteren O-Ring 41 dicht an den Druckraum 37 angeschlossen ist und mit einem Rückschlagventil (nicht im Detail dargestellt) versehen ist, über welches eine Gaspatrone oder ein Adapter 42 zum Anschluss einer Druckgasleitung mit der Spritze 1 verbunden ist.

Im Betrieb wird der notwendige Hydraulikdruck zum Vorschub des Vorschubkolbens 7 vom Druckraum 37 erzeugt. Der Trennkolben 35 trennt dabei das Gasvolumen (unter Druck über den Adapter 42 in den Druckraum 37 gefüllt) vom Hydrauliköl in der zweiten Hydraulikkammer 19. Über die Steuerbohrung 20 kann das unter Druck stehende Hydrauliköl zum Ventilschieber 21 gelangen. Der Ventilschieber wird über die Druckfeder 33 im Ruhezustand verschlossen. Die Druckfeder ist über einen Gewindedeckel (nicht beziffert, im Gehäuse 2 dem Ventilschieber 21 auf der gegenüberliegenden Seite in dessen Verlängerung angeordnet) vorgespannt, um die notwendige Dichtkraft an der Steuerbohrung 20 zu erzeugen. Das Ventil ist ein 2/2-Wege-Sitzventil mit proportionaler Charakteristik, somit kann abhängig vom Ventilhub der Volumenstrom und damit die Ausfuhrgeschwindigkeit des Vorschubkolbens 7 gesteuert werden.

Um den Vorschubkolben 7 auszufahren, muss der Tasterarm 23 bevorzugt an dessen Tastfläche 25 zum Gehäuse 2 der Spritze 1 hin betätigt werden. Dies verschiebt zwangsweise den Ventilschieber 21 entlang seiner Bewegungsrichtung 22 gegen die Druckfeder 33 in das Gehäuse 2 der Spritze 1 hinein. Der freigegebene Strömungsquerschnitt kann nun vom Hydrauliköl von der zweiten Hydraulikkammer 19 zur ersten Hydraulikkammer 16 hin durchflossen werden. Somit wird der Kolbenboden 15 des Vorschubkolbens 7 mit Druck beaufschlagt, so dass der Vorschubkolben 7 ausfährt.

Liegt über das Gewebe, in welches die Spritze 1 eingestochen ist, eine Gegenkraft am Vorschubkolben 7 an, so steigt der Druck in beiden Hydraulikkammern, auf Grund der hydraulischen Verluste an der Steuerbohrung 20 jedoch insbesondere in der ersten Hydraulikkammer 16. Dieser Druck wirkt auf den Fuß 27 des Ventilschiebers 21 in Schließrichtung des Ventils. Der behandelnde Arzt spürt über den Taster 24 den veränderten Druck und muss entsprechend stärker auf den Taster 24 drücken, damit der Ventilschieber 21 in der geöffneten Position und damit das Ventil der Steuerbohrung 20 geöffnet bleibt.

Zusätzlich zu dieser Drucksensierung ist eine optische Druckanzeige über den Indexkolben 28 vorhanden, die den Zylinderdruck und damit die Kolbenkraft - welche als Maß für den Injektionsdruck angesehen werden kann - anzeigt. Dabei fungiert der Indexkolben 28 als kleiner einfachwirkender Hydraulikzylinder, an dessen Kolben drei Nuten zur Anzeige der Druckstärke eingebracht sind. Der Kolben 28 wird von der Feder im Ruhezustand im Gehäuse 2 gehalten (dieser Zustand ist hier nicht dargestellt). Bei ansteigendem Hydraulikdruck in der ersten Hydraulikkammer entsteht über den Fuß 30 des Indexkolbens 28 eine Kraft, welche der Federvorspannung entgegenwirkt und den Indexkolben 28 entsprechend der hydraulischen Druckverhältnisse in der ersten Hydraulikkammer 16 entlang der Bewegungsrichtung 29 aus dem Gehäuse 2 der Spritze 1 mehr oder weniger weit ausfährt. Über die Nuten am Kolben 28 kann dann der Druck abgelesen werden.

Ist der Vorschubkolben 7 komplett ausgefahren und somit die Glascarpule 5 entleert, muss für die nächste Behandlung eine neue Glascarpule in die Carpulenhülse 4 eingelegt werden. Dazu wird die Carpulenhülse 4 über den Bajonettverschluss vom Gehäuse 2 der Spritze 1 getrennt, die Carpule ausgewechselt und die Carpulenhülse 4 anschließend wieder über den Bajonettverschluss mit dem Vorschubzylinder 8 verbunden.

Zusätzlich muss der Vorschubkolben 7 wieder in seine innere Endposition gebracht werden. Dazu muss die Spritze 1 in eine Ladestation eingelegt werden und über einen Hebelmechanismus der Vorschubkolben 7 zurückgeschoben werden. Hierzu betätigt die Ladestation den Tasterarm 23 und öffnet das Ventil der Steuerbohrung 20, damit eine Verbindung zwischen der ersten 16 und der zweiten Hydraulikkammer 19 hergestellt wird. Der Trennkolben 35 bewegt sich beim Rückschieben des Vorschubkolbens 7 zur inneren Endposition zurück und spannt das Gasvolumen im Druckraum 37 wieder auf den ursprünglichen Druck.

Zum nach mehrfachem Gebrauch eventuell notwendigen Nachfüllen oder auch zum Erstbefüllen der Spritze mit dem Federgas zeigt die Figur den Fülladapter 42. Über diesen kann der Druckraum 37 mit Stickstoff unter Druck befüllt werden. Das Rückschlagventil am O-Ring 41 verhindert dabei ein Ausströmen des Gases, wenn der Adapter abgeschraubt wird.

## Patentansprüche

1. Anästhesiespritze (1) mit einer ersten (16) und einer zweiten Hydraulikkammer (19), wobei die zweite Hydraulikkammer (19) rückwärtig der ersten Hydraulikkammer (16) vorgesehen und widerstandsregulierbar mit dieser verbunden ist und wobei in einem Carpulenraum (3) ein längs verschieblicher Vorschubkolben (7) mit einer Druckplatte (15) vorgesehen ist, welche an die erste Hydraulikkammer (16) angeschlossen ist, sowie mit einem Ventilschieber (21), welcher eine Öffnung einer Steuerbohrung (20) zwischen der ersten (16) und der zweiten (19) Hydraulikkammer verschließen oder öffnen kann, **dadurch gekennzeichnet, dass** der Ventilschieber (21) eine Ventilschieberdruckplatte (27) aufweist, welche an die erste Hydraulikkammer (16) angeschlossen ist.

2. Anästhesiespritze nach Anspruch 1, ***dadurch gekennzeichnet, dass*** der Trennkolben einen Druckraum vergrößerbar verschieblich gelagert ist, wobei eine den Druckraum vergrößernde Verschiebung des Trennkolbens eine Verkleinerung der zweiten Hydraulikkammer bewirkt.

3. Anästhesiespritze nach Anspruch 2, ***dadurch gekennzeichnet, dass*** bei einer Vergrößerung des Druckraums durch Verschiebung des Trennkolbens die zweite Hydraulikkammer um ein gleiches Maß verkleinert wird.

4. Anästhesiespritze nach Anspruch 3, ***dadurch gekennzeichnet, dass*** der Ventilschieber mit einem Stirnelement in die erste Hydraulikkammer hineinragt.

5. Anästhesiespritze nach einem der Ansprüche 3 oder 4, ***dadurch gekennzeichnet, dass*** eine Bewegungsachse eines Ventilschiebers senkrecht zu einer Längserstreckungsrichtung der Spritze angeordnet ist.

6. Anästhesiespritze nach einem der Ansprüche 3 bis 5, ***dadurch gekennzeichnet, dass*** ein Ventilschieber mit einer eine Steuerbohrung schließenden Vorspannkraft beaufschlagt ist.

7. Anästhesiespritze nach einem der vorhergehenden Ansprüche, ***gekennzeichnet durch*** einen Indexkolben, welcher an die erste Hydraulikkammer angeschlossen ist.

8. Anästhesiespritze nach Anspruch 7, ***dadurch gekennzeichnet, dass*** der Indexkolben mit einem Fuß in die erste Hydraulikkammer hineinragt.

9. Anästhesiespritze nach Anspruch 7 oder 8, ***dadurch gekennzeichnet, dass*** der Indexkolben zumindest teilweise aus dem Gehäuse der Spritze hinausragend verschieblich gelagert ist, wobei ein Austrittsbegrenzer vorgesehen ist.

## Claims

1. An anaesthetic syringe (1) with a first (16) and a second hydraulic chamber (19), said second hydraulic chamber (19) being provided at the rear of said first hydraulic chamber (16) to which it is connected for resistance regulation, a longitudinally slidable feed piston (7) with a pressure plate (15) being provided in a syringe cylinder (3), said pressure plate (15) being connected to said first hydraulic chamber (16), and with a slide valve (21) capable of closing or opening an opening of a control hole (20) between said first (16) and said second (19) hydraulic chamber, **characterized in that** said slide valve (21) comprises a slide valve pressure plate (27) that is connected to said first hydraulic chamber (16).

2. The anaesthetic syringe as set forth in claim 1, ***characterized in that*** the dividing piston is slidably carried for enlarging a pressure volume, a displacement of said dividing piston that enlarges the pressure volume causing the second hydraulic chamber to become smaller.

3. The anaesthetic syringe as set forth in claim 2, ***characterized in that**,* as the size of the pressure volume increases as a result of the displacement of the dividing piston, the second hydraulic chamber becomes smaller by the same amount.

4. The anaesthetic syringe as set forth in claim 3, ***characterized in that*** the slide valve has an end element that projects into the first hydraulic chamber.

5. The anaesthetic syringe as set forth in any one of the claims 3 or 4, ***characterized in that*** an axis of movement of a slide valve is disposed at right angles to the direction in which the syringe extends longitudinally.

6. The anaesthetic syringe as set forth in any one of the claims 3 through 5, ***characterized in that*** a slide valve is biased by a pre-bias force that closes a control hole.

7. The anaesthetic syringe as set forth in any one of the previous claims, ***characterized by*** an index piston that is connected to the first hydraulic chamber.

8. The anaesthetic syringe as set forth in claim 7, ***characterized in that*** the index piston has one foot projecting into the first hydraulic chamber.

9. The anaesthetic syringe as set forth in claim 7 or 8, ***characterized in that*** the index piston is slidably carried so as to protrude at least partially from the housing of the syringe, an end-point limiter being provided.

## Revendications

1. Seringue pour anesthésie (1) avec une première (16) et une deuxième chambre hydraulique (19), la deuxième chambre hydraulique (19) étant prévue derrière la première chambre hydraulique (16) et étant reliée à celle-ci de manière à pouvoir en réguler la résistance, et un piston poussoir (7) mobile en coulissement dans le sens de la longueur avec un plateau presseur (15) étant prévu dans un cylindre de seringue (3), ce plateau presseur étant raccordé à la première chambre hydraulique (16), ainsi qu'avec un obturateur (21) capable de fermer ou d'ouvrir une ouverture d'un trou de contrôle (20) entre la première (16) et la deuxième (19) chambre hydraulique, **caractérisée en ce que** l'obturateur (21) comporte une plaque de pression (27) qui est raccordée à la première chambre hydraulique (16).

2. Seringue pour anesthésie selon la revendication 1, ***caractérisée en ce que*** le piston de séparation est monté mobile en coulissement de manière à agrandir un volume de pression, un coulissement du piston de séparation agrandissant le volume de pression entraînant une réduction de la taille de la deuxième chambre hydraulique.

3. Seringue pour anesthésie selon la revendication 2, ***caractérisée en ce que**,* si le volume de pression s'agrandit du fait du coulissement du piston de séparation, la taille de la deuxième chambre hydraulique diminue du même montant.

4. Seringue pour anesthésie selon la revendication 3, ***caractérisée en ce que*** l'obturateur a un élément d'extrémité qui fait saillie dans la première chambre hydraulique.

5. Seringue pour anesthésie selon l'une quelconque des revendications 3 ou 4, ***caractérisée en ce qu'**un* axe de mouvement d'un obturateur est disposé à angle droit par rapport au sens de la longueur sur lequel s'étend la seringue.

6. Seringue pour anesthésie selon l'une quelconque des revendications 3 à 5, ***caractérisée en ce qu'**une* force de précontrainte fermant un trou de contrôle est appliquée sur l'obturateur.

7. Seringue pour anesthésie selon l'une quelconque des revendications précédentes, ***caractérisée par*** un piston formant index qui est raccordé à la première chambre hydraulique.

8. Seringue pour anesthésie selon la revendication 7, ***caractérisée en ce que*** le piston formant index a un pied qui fait saillie dans la première chambre hydraulique.

9. Seringue pour anesthésie selon la revendication 7 ou 8, ***caractérisée en ce que*** le piston formant index est monté mobile en coulissement de manière à faire saillie du moins partiellement hors du boîtier de la seringue, un limiteur de course étant prévu.
